# EUROPEAN PATENT APPLICATION

(11) **EP 3 656 414 A1**
(43) Date of publication of application: **27.05.2020**
(21) Application number: 18207330.4
(22) Date of filing: 20.11.2018
(51) Int. Cl.: A61M 1/36

(54) **A VENOUS CANNULA FOR USE IN A CARDIOPULMONARY BYPASS OR EXTRA-CORPOREAL MEMBRANE OXYGENATION SYSTEM**

(71) Applicant: Universiteit Maastricht, 6211 LK Maastricht (NL); Academisch ziekenhuis Maastricht, 6229 HX Maastricht (NL)
(72) Inventor: LORUSSO, Roberto, 6229 HX MAASTRICHT (NL)
(74) Representative: Algemeen Octrooi- en Merkenbureau B.V.

(57) **Abstract**

The invention relates a venous cannula for use in a cardiopulmonary bypass system or during extra-corporeal life support (ECLS)/membrane oxygenation (ECMO), the venous cannula being composed of an elongated, flexible tubular body having a proximal tip end for insertion in a vein towards the heart of a human or animal subject, and a distal end for connection to the cardiopulmonary bypass/ECLS/ECMO system, the elongated, flexible tubular body being equipped with a drainage capacity by volume for draining blood from the vein, which drainage capacity varies between the proximal tip end and the distal end.

## Description

### FIELD OF THE INVENTION

The invention relates to a venous cannula for use in a cardiopulmonary bypass or extra-corporeal membrane oxygenation system.

### BACKGROUND OF THE INVENTION

Extra Corporeal Life Support (ECLS), named also as Extra Corporeal Membrane Oxygenation (ECMO) consists of the application of principles linked to the cardiopulmonary bypass machine used in routine cardiac surgery. In cardiac surgical procedures an extracorporeal cardiopulmonary bypass system is utilized in order to mechanically perform the functions normally conducted by the heart and lungs. This apparatus replicates the heart and lung functions by draining partial or almost the entire patient venous blood volume, being depleted in oxygen and rich in carbon dioxide, outside the body and, after appropriate blood gas exchange in an artificial membrane lung, reinjecting the oxygenated and decarboxylated blood into the patient's vessel (arterial system), so as to allow the lungs and heart to reduce their functional work and allow recovery from insults.

ECLS was initiated in the early 1970s, but has recently regained a worldwide interest following the H1N1 pandemia, and by the growing interest about its role in the cardiogenic shock and cardiac arrest rescues. The ECLS/ECMO circuits consist of one line for the blood drainage from the venous vasculature towards the ECLS pump, then a membrane lung to enhance blood gas exchange, and reinfused into the arterial or venous vessels depending on the kind of support (arterial in case of heart or heart/lung compromise, or venous in case of isolated lung dysfunction). Multiple cannulas, either for drainage or reperfusion, may be used at time depending on patient's status and conditions.

The venous cannula, which is the dedicated and special catheter implanted in the venous system, either through a direct or a percutaneous approach, towards the heart of the human or animal subject under cardiac surgery to drain the blood out from the body towards the ECLS apparatus, needs to have a sufficient drainage capacity to drain the venous blood from the venous system and towards the ECLS apparatus. Drainage of blood from the venous vessel through the venous cannula towards the ECLS apparatus is performed under active or passive aspiration/drainage in relation to which type of pump (centrifugal, roller or other) is utilized.

Due to the configuration of the venous cannulas presently known in the art, the aspirational operation of the centrifugal pump creates an ineffective blood volume suction across the longitudinal orientation of the venous cannula. In particular, during operation of ECLS apparatus major volume suction phenomena are created closer to the aspiration side of the centrifugal pump, with a remarkable reduction of blood volume suction towards the free tip of the cannula.

This results in that the majority (almost 50%) of the negative pressure (and, therefore theoretically, of the total blood volume) is exerted on the distal side of the venous cannula closest to the centrifugal pump of the ECLS apparatus with a further reduction of negative pressure and thus drainage capacity towards the tip of the cannula near the heart.

This volume suction phenomenon will result in a very limited blood volume drained from the venous vasculature towards the ECLS apparatus near the free tip of the venous cannula, which is usually placed in the vein near the heart or even in the right atrium of the heart up to the superior vena cava. This induces two important and negative phenomena: first, the venous blood coming from the upper part of the body (that is from the superior vena cava to the right atrium) is limitedly drained or not drained at all, and secondly, as the highest volume suction is exerted on the distal part of the venous cannula located usually in the inferior vena cava and closest to the centrifugal pump of the ECSL apparatus, this high negative pressure may cause a full collapse of the venous walls of the inferior vena cava onto the venous cannula, if too high, like more than 70-80 mmHg or with a hypovolemic patient with a collapsed inferior vena cava and obstruction of the cannula holes due to the adhesion of the venous wall to the draining holes.

This might adversely affect the drainage capacity (suction surface) and further reduce the drained volume. This phenomenon is usually treated by reducing the negative pressure exerted by the active ECLS drainage, or by administering more fluids and hence volume to the patient, thereby creating additional problem like reduce pump flow to the patient or excessive fluid administration with associated complications, like lung or global body overfilling.

### DESCRIPTION OF THE INVENTION

The invention aims to provide a solution for the above identified problems, allowing an improved drainage of venous blood from the vein and/or heart of the human or animal subject towards the ECLS apparatus. Furthermore, this invention might provide better cardio-pulmonary bypass management also in routine cardiac surgery operations.

According to the invention, a venous cannula for use in a cardiopulmonary bypass system is proposed, the venous cannula being composed of an elongated, flexible tubular body having a proximal tip end for insertion in a vein towards or in the heart of a human or animal subject and a distal end for connection to the cardiopulmonary bypass system, the elongated, flexible tubular body being equipped with a drainage capacity by volume for draining blood from the vein, which drainage capacity varies between the proximal tip end and the distal end.

In particular the drainage capacity of the venous cannula is larger at the proximal tip end than at the distal end.

Herewith, the drainage of the venous blood directly coming from the upper part of the body (that is from the superior vena cava to the right atrium) is promoted furthermore, although the highest volume suction is still exerted on the distal part of the venous cannula located usually in the inferior vena cava and closest to the centrifugal pump of the ECSL apparatus, this high negative pressure does not result in the highest drainage of blood near the distal part of the venous cannula and thus a full collapse of the venous walls of the inferior vena cava onto the venous cannula is prevented.

In a particular example of the venous cannula the drainage capacity of the venous cannula monotonically decreases seen in the direction of the proximal tip end towards the distal end, whereas in another example the drainage capacity of the venous cannula decreases linearly seen in the direction of the proximal tip end towards the distal end.

In an particular advantageous example the elongated tubular body of the venous cannula is made of a porous material, the porosity of said porous material decreases seen in the direction of the proximal tip end towards the distal end.

In another advantageous example the elongated, tubular body of the venous cannula is provided with multiple drainage holes distributed through and along the circumference of the tubular body and in particular the distribution of the multiple drainage holes varies between the proximal tip end and the distal end, thus effectively allowing to vary the drainage capacity.

In an example allowing an effective varying drainage capacity the number of drainage holes is larger near the proximal tip end than near the distal end.

In an example allowing an effective varying drainage capacity the drainage sizes of the multiple drainage holes vary between the proximal tip end and the distal end.

In particular the drainage sizes of the multiple drainage holes are larger near the proximal tip end than near the distal end.

In yet another advantageous example of a venous cannula with a varying drainage capacity, said multiple drainage holes are distributed in two or more sub-groups along the circumference of said tubular body, each sub-group having a different number of drainage holes and/or each sub-group having drainage holes with a same drainage size, which differs from the drainage size of the drainage holes of another sub-group.

In particular the said multiple drainage holes are distributed in two till eight sub-groups, with three till six holes per subgroup, wherein, seen in the direction of the proximal, free tip end 22a towards the distal end 20b of the cannula 20, the diameter of a drainage hole 22 of each group is 5.5 mm in sub-group 22-1, 5.5 or 5 mm in sub-group 22-2, 5 or 4.5 mm in sub-group 22-3, 4.5 or 4 mm in sub-group 22-4, 4 or 3 mm in sub-group 22-5, 3 or 2.5 mm in sub-group 22-6, 2.5 or 2 mm in group 22-7 and 2 mm in sub-group 22-8.

In case collapse of the venous wall towards the cannula will occur, the higher drainage capacity (higher number of holes, or larger hole diameters present towards the cannula tip in the heart) will compensate and guarantee adequate blood drainage.

### BRIEF DESCRIPTION OF THE DRAWINGS

The invention will now be described in more details in reference to accompanying drawings, which drawings show in:
Figure 1A a schematic embodiment of a human patient undergoing veno-venous (V-V) extracorporeal life support;
Figure 1B a schematic embodiment of a human patient undergoing veno-arterial (V-A) extracorporeal life support;
Figure 2 a schematic detail of an embodiment of a conventional and currently adopted venous cannula for use in extracorporeal life support applications;
Figure 3-6 a schematic detail of several embodiments of a venous cannula according to the invention for use in extracorporeal life support applications.

### DETAILED DESCRIPTION OF THE INVENTION

For a better understanding of the invention like parts in the drawings are to be denoted with like reference numerals.

In the detailed description below as well as in the claims various parts are denoted with the classification "proximal" and "distal". These classifications are to be considered in relation to the location of the heart of the human or animal subject in which the invention is to be used. Hence the classification "proximal" is to be understood as meaning "closest to the heart" or "in a direction towards the heart". Similarly "distal" is to be understood as meaning "farthest from the heart" or "in a direction away from the heart", that is towards the ECLS system (pump or oxygenator).

Extracorporeal life support (ECLS), also known as extracorporeal membrane oxygenation (ECMO), is an extracorporeal technique of providing prolonged cardiac and respiratory support to persons whose heart and lungs are unable to provide an adequate amount of gas exchange and/or perfusion to sustain life. The technology for ECMO is largely derived from cardiopulmonary bypass, which provides shorter-term support. This intervention has mostly been used on children, but it is seeing more use in adults with cardiac and respiratory failure.

ECMO works by removing blood from the person's body and artificially removing the carbon dioxide and oxygenating red blood cells. Generally, it is used either post-cardiopulmonary bypass or in late stage treatment of a person with profound heart and/or lung failure, although it is now seeing use as a treatment for cardiac arrest in certain centers, allowing treatment of the underlying cause of arrest while circulation and oxygenation are supported.

ECLS or ECMO consists of the application of principles linked to the cardiopulmonary bypass machine used in routine cardiac surgery. In cardiac surgical procedures an extracorporeal cardiopulmonary bypass apparatus or system is utilized in order to mechanically perform the functions normally conducted by the heart and lungs. This apparatus replicates the heart and lung functions by draining partial or almost the entire patient venous blood volume, being depleted in oxygen and rich in carbon dioxide, outside the body and, after appropriate blood gas exchange in an artificial membrane lung, reinjecting the oxygenated and decarboxylated blood into the patient's vessel (arterial system), so as to allow the lungs and heart to reduce their functional work and allow recovery from insults.

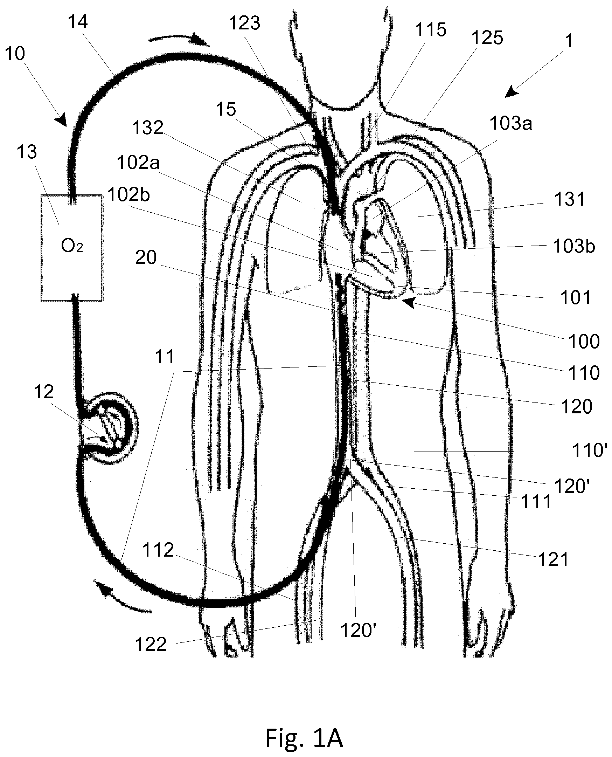

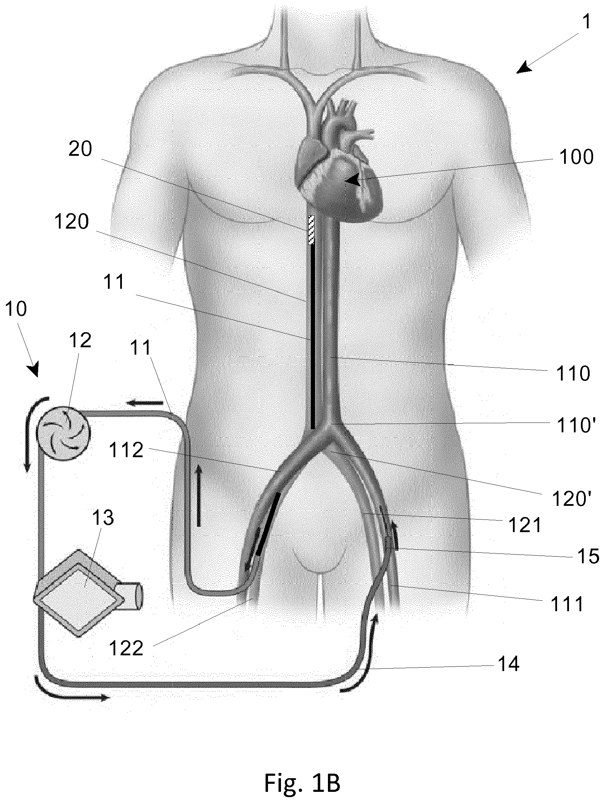

An application of ECLS or ECMO implementing an extracorporeal cardiopulmonary bypass system 10 is depicted in Figure 1A, depicting a front view of the thorax and abdomen of a human being 1. It is noted that the left side of the Figure 1A (but also of Figure 1B) corresponds with the right side in the human body and vice versa.

The front view of the human being 1 depicted in Figure 1A shows the heart 100, as well as the left and right lungs 131 and 132. The human heart 100 is divided into four chambers, named the upper left atrium 103a and the upper right atrium 102a and the lower left ventricle 103b and the lower right ventricle 102b. The upper right atrium 102a receives predominately deoxygenated blood from the body's two major veins, the inferior vena cava 120 and the superior vena cava 123. The upper right atrium 102a is connected to the lower right ventricle 102b via the tricuspid heart valve and from the lower right ventricle 102b blood is pumped via the pulmonary heart valve towards the lungs 131-132 via the pulmonary arteries 115.

From the lungs 131-132 oxygenated blood is returned via the pulmonary veins 125 and enters the upper left atrium 103a. Via the mitral heart valve said blood enters the lower left ventricle 103b, from which the oxygenated blood is pumped via the aortic heart valve into the aorta 110 and the circulatory system of the human vascular system. At the level of the fourth (or fifth) lumbar vertebra the aorta 110 divides via the aortic bifurcation 110' into the left femoral artery 111 and the right femoral artery 112, which further distribute oxygenated blood to all parts of the left and right lower extremities through the systemic circulation.

Reference numerals 121 and 122 depict the left and right femoral veins, which carry deoxygenated blood from the left and right lower extremities respectively tissues back to the heart 100. The left and right femoral veins 121 and 122 join at the level of the fourth (or fifth) lumbar vertebra into the inferior vena cava bifurcation 120', and form the inferior vena cava 120.

The ECLS/ECMO bypass apparatus or system 10 consist of a drainage line 11 for the blood drainage from the venous vasculature towards the ECLS pump 12. Pump 12 be a centrifugal pump or roll-cell pump which pump operates under the principle of aspiration. The pump 12 pumps the blood towards an artificial membrane lung 13 to enhance blood gas exchange, and the oxygenated and decarboxylated blood is then reinjected via reinjection line 14 into the arterial or venous vessels depending on the kind of support (arterial in case of heart or heart/lung compromise, or venous in case of isolated lung dysfunction).

Figure 1A depicts a human patient 1 undergoing veno-venous (V-V) extracorporeal life support, with a venous cannula 20 together with the drainage line 11 being inserted into the inferior vena cava 120 via one of the left or right femoral veins 121-122. Usually the venous cannula 20 is inserted with its proximal, free tip end until in the upper right atrium 102a of the heart 100 and is the dedicated and special catheter to drain the blood out from the body 1 towards the ECLS apparatus 10. Oxygenated and decarboxylated blood is reinjected via line 14 using a catheter 15 being inserted into the right or left pulmonary artery 115.

Figure 1B depicts an alternative application with a human patient 1 undergoing veno-arterial (V-A) extracorporeal life support with the venous cannula 20 being inserted via the right femoral vein 122 with its proximal free tip end near or until in the upper right atrium 102a of the heart 100 and oxygenated and decarboxylated blood is reinjected via line 14 using the catheter 15 being inserted into the left femoral artery 111.

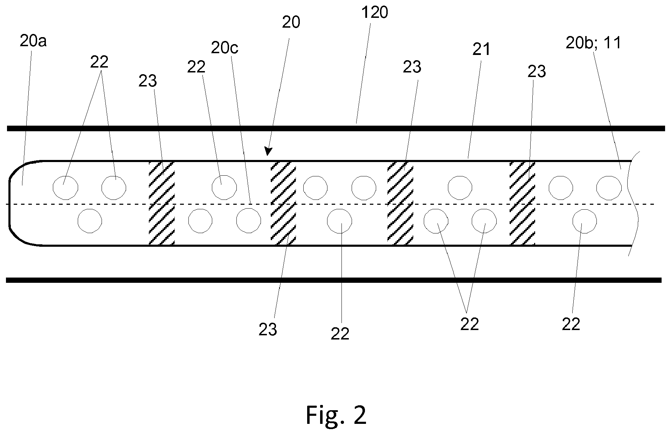

An example of a venous drainage cannula or catheter 20 according to the state of the art is depicted in more detail in Figure 2. For the sake of clarity the dimensions and certain elements of the cannula 20 are exaggerated. The venous cannula 20 is composed of an elongated, flexible hollow tubular body 21 having a proximal tip end 20a for insertion in a vein (here the inferior vena cava 120 as depicted in Figure 1A) towards the heart 100 of a patient 1. The distal end 20b of the venous cannula 20 is provided with the line 11 (see Figure 1A and 1B) for connection to the cardiopulmonary bypass system 10.

The elongated, flexible hollow tubular body 21 is provided with multiple drainage holes 22, which are evenly distributed along and through the circumference of the elongated, hollow flexible tubular body. The drainage holes 22 serve to drain venous blood from the vein 120 into the hollow flexible tubular body towards the line 11 and the cardiopulmonary bypass apparatus 10 due to the aspiration of the centrifugal pump 12.

The venous drainage cannula or catheter 20 needs to have a sufficient drainage capacity to drain the venous blood from the vein 120 and towards the ECLS apparatus. Due to the configuration of the venous cannulas 20 presently known in the art the aspirational operation of the centrifugal pump 12 creates an ineffective blood volume suction across the longitudinal orientation 20c of the venous cannula 20.

In particular during operation of the ECLS apparatus 10 major volume suction phenomena are created closer to the aspiration side (the distal end side 20b) of the centrifugal pump 12, with a remarkable reduction of blood volume suction towards the free tip 20a of the venous cannula 20. This results in that the majority (almost 50%) of the negative pressure (and, therefore theoretically, of the total blood volume) is exerted on the distal side 20b of the venous cannula 20 closest to the centrifugal pump 12 of the ECLS apparatus 10 with a further reduction of negative pressure and thus drainage capacity towards the tip 20a of the venous cannula 20 near the heart 100.

This volume suction phenomenon will result in a very limited blood volume drained from the venous vasculature 120 towards the ECLS apparatus 10 near the free tip 20a of the venous cannula 20, which is inserted in the vein 120 near the heart 100 or even in the upper right atrium 102a of the heart 100. This induces two important and negative phenomena: first, the venous blood coming from the upper part of the body (that is from the superior vena cava 123 to the upper right atrium 102a) is limitedly drained or not drained at all and secondly, as the highest volume suction is exerted on the distal end part 20b of the venous cannula 20 located usually in the inferior vena cava 120 and closest to the centrifugal pump 12 of the ECSL apparatus 10, this high negative pressure may cause a full collapse of the venous walls of the inferior vena cava 120 onto the venous cannula 20, if too high like more than 70-80 mmHg or with a hypovolemic patient with a collapsed inferior vena cava 120.

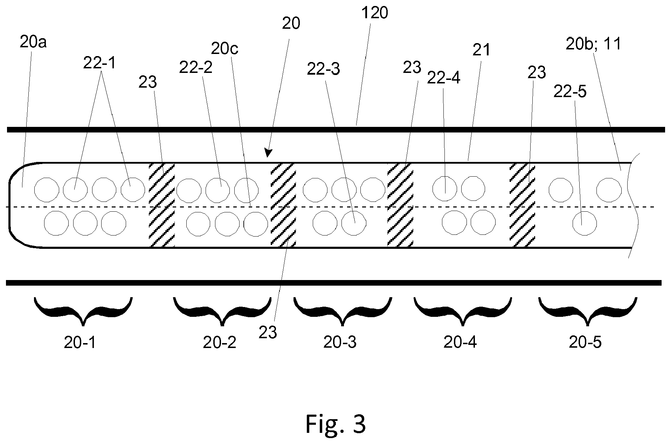

As a solution for this problem several embodiments of venous cannulas according to the invention as depicted in Figures 3-6 having an improved drainage capacity compared to the venous cannula 20 as depicted in Figure 2. In all embodiments according to the invention the venous cannulas 20 of Figures 3-6 are equipped with a drainage capacity by volume for draining blood from the vein, which drainage capacity varies between the proximal tip end 20a and the distal end 20b of the cannula 20 and in particular the drainage capacity of the venous cannula 20 is larger at the proximal tip end 20a than at the distal end 20b, seen in the longitudinal direction 20c of the cannula 20.

Herewith drainage of the venous blood directly coming from the upper part of the body (that is from the superior vena cava 123 to the upper right atrium 102a) is promoted furthermore, although the highest volume suction is still exerted on the distal part 20b of the venous cannula 20 located usually in the inferior vena cava 120 and closest to the centrifugal pump 12 of the ECSL apparatus 10, this high negative pressure does not result in the highest drainage of blood near the distal part 20b of the venous cannula 20 and thus a full collapse of the venous walls of the vein (inferior vena cava) 120 onto the venous cannula 20 is prevented.

As shown in the several embodiment of Figures 3-6 the drainage capacity of the venous cannula 20 monotonically decreases seen in the direction of the proximal tip end 20a towards the distal end 20b, in particular decreases linearly seen in the direction of the proximal tip end 20a towards the distal end 20b along the longitudinal direction 20c of the cannula 20.

The decrease of the drainage capacity of the venous cannula 20 is effected by a varying distribution (in size and/or dimensions) of the multiple drainage holes 22 between the proximal tip end 20a and the distal end 20b.

As shown in the embodiment of Figure 3 the number of drainage holes 22 is larger near the proximal tip end 20a than near the distal end 20b.

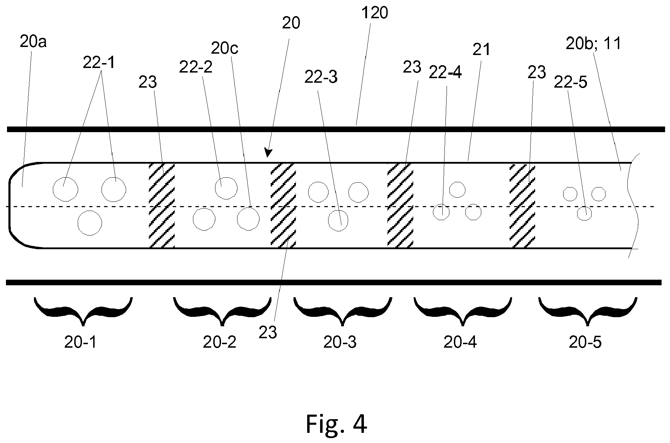

As shown in the embodiment of Figure 4 the drainage sizes of the multiple drainage holes 22 vary between the proximal tip end 20a and the distal end 20b with the drainage sizes of the multiple drainage holes 22-1 near the proximal free tip end 20a being larger than the drainage sizes of the multiple drainage holes 22-5 near the distal end 20b.

As depicted in the several embodiments of Figures 3-5 the multiple drainage holes 22 are distributed in two or more sub-groups along the circumference of said elongated tubular body 21, wherein each sub-group is marked the index (22-n). In the Figures 3-5 five sub-groups 22-n of drainage holes 22 are shown for explanation of the varying drainage capacity by volume for draining blood from the vein between the proximal tip end 20a and the distal end 20b of the cannula 20. The five sub-groups are marked 22-1 (nearest to the proximal tip end 20a and farthest from the distal end 20b of the venous cannula 20), 22-2, 22-3, 22-4, and 22-5 (farthest from the proximal tip end 20a and nearest to the distal end 20b of the venous cannula 20).

However it is noted that the invention is not limited to five sub-groups, but that also a lesser amount of areas (two, three or four) or even a larger amount of porosity areas (six, seven, eighth, or even more than ten) can be implemented depending on the length of the venous cannula 20 or the desired drainage capacity.

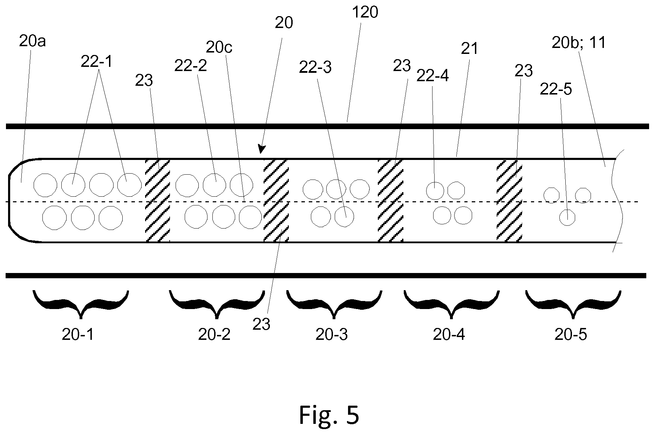

Depending of the physique of the patient or the desired drainage capacity or the intended ECMO application each sub-group 22-1 till 22-5 (or in more general 22-n) can have a different number of drainage holes 22 but with same drainage sizes (the embodiment of Figure 3), or each sub-group 22-1 till 22-n can have drainage holes with a same number and a same drainage size for each sub-group, but with a drainage size (e.g. diameter) which differs from the drainage size of the drainage holes of another sub-group (the embodiment of Figure 4). Alternatively each sub-group 22-1 till 22-n can have drainage holes with a same drainage size for each sub-group, but with a number and a drainage size (e.g. diameter) which differs from the number and drainage size of the drainage holes of another sub-group (the embodiment of Figure 5).

As shown in the embodiments of Figures 3-5 the multiple drainage holes 22 can be distributed in two till ten sub-groups, with three till six or seven holes per subgroup, evenly distributed over the circumference of the elongated tubular body 21.

In particular, seen in the direction of the proximal, free tip end 22a towards the distal end 20b of the cannula 20, the diameter of a drainage hole 22 of each group is 5.5 mm in sub-group 22-1, 5.5 or 5 mm in sub-group 22-2, 5 or 4.5 mm in sub-group 22-3, 4.5 or 4 mm in sub-group 22-4, 4 or 3 mm in sub-group 22-5, 3 or 2.5 mm in sub-group 22-6, 2.5 or 2 mm in group 22-7 and 2 mm in sub-group 22-8.

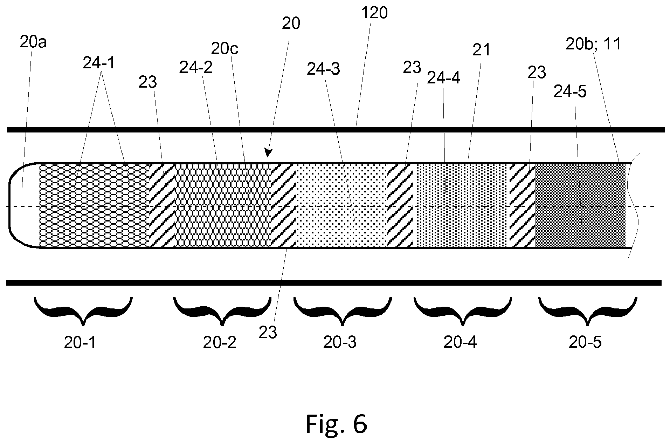

An particular advantageous example is depicted in Figure 6, wherein the elongated tubular hollow body 21 of the venous cannula 20 is made of a porous material 24, wherein the porosity of said porous material 24 decreases seen in the direction of the proximal tip end 20a towards the distal end 20b along the longitudinal direction 20c of the cannula 20. In Figure 6 the porous material 24 of the elongated tubular hollow body 21 monotonically decreases seen in the direction of the proximal tip end 20a towards the distal end 20b and is composed of several porosity areas indicated with reference numerals 24-1 till 24-5, the porosity of area 24-1 closest to the proximal tip end 20a being the highest, and the porosity of each subsequent porosity area 24-2, 24-3, 24-4, 24-5 further decreases towards the distal end 20b.

The porosity of each area 24-1 till 24-5 can be defined by the material properties or pore size or pore density, which is largest near the proximal, free tip end 20a and decreases towards the distal end 20b of the venous cannula 20.

Also in this embodiment, five porosity areas are depicted for explanation of the varying drainage capacity by volume for draining blood from the vein between the proximal tip end 20a and the distal end 20b of the cannula 20. However it is noted that the invention is not limited to five porosity areas, but that also a lesser amount of areas (two, three or four) or even a larger amount of porosity areas (six, seven, eighth, or even more than ten) can be implemented depending on the physique of the patient or the desired drainage capacity or the intended ECMO application or the length of the venous cannula 20. It should be clear that the drainage capacity (the porosity) of the several porosity areas 24-1 till 24-n (with n the total number of areas) of the venous cannula 20 decreases seen in the direction of the proximal tip end 20a towards the distal end 20b.

Reference numeral 23 denotes a reinforcement / flexible element part or material worked at several, distinct locations in the elongated, tubular body 21 of the venous cannula 20 providing flexibility and bending of the cannula during insertion into the vein.

It is noted that the drainage holes 22 are depicted as round holes in the Figures, however it should be noted that also other shapes (oval, etc.) can be implemented as long as the drainage capacity of the venous cannula 20 decreases seen in the direction of the proximal tip end 20a towards the distal end 20b.

### REFERENCE NUMERAL LISTING

- 1: human body (patient)
- 10: extracorporeal cardiopulmonary bypass system
- 11: drainage line
- 12: extracorporeal cardiopulmonary life support pump
- 13: artificial membrane lung
- 14: reinjection line
- 15: reinjection catheter
- 20: venous drainage cannula or catheter
- 20-1..20-n: n number of distinct areas of the venous drainage cannula or catheter
- 20a: proximal, free end tip of venous drainage cannula
- 20b: distal end of venous drainage cannula
- 20c: longitudinal axis of venous drainage cannula
- 21: elongated, flexible tubular body of cannula 20
- 22: drainage hole
- 22-1..22-n: n sub-groups of drainage holes
- 23: reinforcement / flexible element part
- 100: human (living) heart
- 101: exterior region (pericardium / heart tissue wall) of the heart
- 102a: upper right atrium
- 102b: lower right ventricle
- 103a: upper left atrium
- 103b: lower left ventricle
- 110: aorta
- 110': aortic bifurcation
- 111: left femoral artery
- 112: right femoral artery
- 115: pulmonary artery or arteries
- 120: inferior vena cava
- 120': inferior vena cava bifurcation
- 121: left femoral vein
- 122: right femoral vein
- 123: superior vena cava
- 125: pulmonary vein or veins
- 50: venous cannula

## Claims

1. A venous cannula for use in a cardiopulmonary bypass system, the venous cannula being composed of an elongated, flexible tubular body having a proximal tip end for insertion in a vein towards the heart of a human or animal subject and a distal end for connection to the cardiopulmonary bypass system, the elongated, flexible tubular body being equipped with a drainage capacity by volume for draining blood from the vein, which drainage capacity varies between the proximal tip end and the distal end.

2. The venous cannula according to claim 1, wherein the drainage capacity of the venous cannula is larger at the proximal tip end than at the distal end.

3. The venous cannula according to claim 1 or 2, wherein the drainage capacity of the venous cannula monotonically decreases seen in the direction of the proximal tip end towards the distal end.

4. The venous cannula according to claim 3, wherein the drainage capacity of the venous cannula decreases linearly seen in the direction of the proximal tip end towards the distal end.

5. The venous cannula according to any one or more of the claims 1-4, wherein the elongated, tubular body is provided with multiple drainage holes distributed through and along the circumference of the tubular body.

6. The venous cannula according to claim 5, wherein the distribution of the multiple drainage holes varies between the proximal tip end and the distal end.

7. The venous cannula according to claim 6, wherein the number of drainage holes is larger near the proximal tip end than near the distal end.

8. The venous cannula according to claim 5, wherein the drainage sizes of the multiple drainage holes vary between the proximal tip end and the distal end.

9. The venous cannula according to claim 5, wherein the drainage sizes of the multiple drainage holes are larger near the proximal tip end than near the distal end.

10. The venous cannula according to any one or more of the claims 5-9, wherein said multiple drainage holes are distributed in two or more sub-groups along the circumference of said tubular body, each sub-group having a different number of drainage holes and/or each sub-group having drainage holes with a same drainage size, which differs from the drainage size of the drainage holes of another sub-group.

11. The venous cannula according to claim 10, wherein the said multiple drainage holes are distributed in two till eight sub-groups, with three till six holes per subgroup.

12. The venous cannula according to claim 10 or 11, wherein, seen in the direction of the proximal, free tip end 22a towards the distal end 20b of the cannula 20, the diameter of a drainage hole 22 of each group is 5.5 mm in sub-group 22-1, 5.5 or 5 mm in sub-group 22-2, 5 or 4.5 mm in sub-group 22-3, 4.5 or 4 mm in sub-group 22-4, 4 or 3 mm in sub-group 22-5, 3 or 2.5 mm in sub-group 22-6, 2.5 or 2 mm in group 22-7 and 2 mm in sub-group 22-8.
